Europäisches Patentamt

European Patent Office

Office européen des brevets

(19)

(11) Numéro de publication: **0 090 732**
**B1**

(12) **FASCICULE DE BREVET EUROPEEN**

(45) Date de publication du fascicule du brevet:
16.07.86

(51) Int. Cl.⁴: **A 61 K 39/36**

(21) Numéro de dépôt: **83400622.3**

(22) Date de dépôt: **24.03.83**

(54) Nouveau produit protéique, obtention et application comme médicament, en particulier immunorégulateur et antiallergique.

(30) Priorité: **26.03.82 FR 8205241**

(43) Date de publication de la demande:
**05.10.83 Bulletin 83/40**

(45) Mention de la délivrance du brevet:
**16.07.86 Bulletin 86/29**

(84) Etats contractants désignés:
**BE CH DE FR GB LI SE**

(56) Documents cités:
**US - A - 1 613 313**

**BIOLOGICAL ABSTRACTS, vol. 66, no. 71, 1 juillet 1978, page 76, Phila. PA. USA; M.D. TOPPING et al.: "Allergenic activity of fractions of cocksfoot (Dactylis glomerata) pollen"**

(73) Titulaire: **INSTITUT PASTEUR, 28, rue du Docteur Roux, F-75715 Paris Cedex 15 (FR)**

(72) Inventeur: **David, Bernard, 7, rue du 8 mai 1945, F-94350 Villiers-sur-Marne (FR)**
Inventeur: **Peltre, Gabriel, 30, rue des Maronites, F-75020 Paris (FR)**
Inventeur: **Mecheri, Salah, 15, rue du Faubourg Saint-Antoine, F-75011 Paris (FR)**

(74) Mandataire: **Phélip, Bruno et al, c/o Cabinet Harié & Phélip 21, rue de la Rochefoucauld, F-75009 Paris (FR)**

## Description

La présente invention concerne un nouveau produit consistant en une protéine. Elle est également relative à un procédé permettant l'isolement d'une telle protéine. L'invention a encore pour objet l'application de ce nouveau produit comme médicament, en particulier immunorégulateur et antiallergique. Sous l'un de ses aspects, l'invention appartient donc au domaine de l'immunologie ainsi que de l'allergie.

On sait que les manifestations allergiques sont très fréquentes et qu'elles sont dues à un grand nombre de facteurs. Les maladies allergiques sont tenaces, en raison même de la présence constante ou occasionnelle des facteurs déclenchant les réactions allergiques. Il est donc souhaitable d'augmenter les réponses immunitaires de l'organisme pour lui permettre de mieux résister aux agressions extérieures et notamment aux facteurs responsabels des réactions allergiques.

Parmi les causes d'allergie, on cite souvent des pollens des plantes. L'homme de l'art pourra à cet égard se reporter à l'article très documenté de Raymonde BARREAU et Jean-René MALLET dans «Journal de Médecine de Bordeaux», 1958, 135, 1379–1389. Cette référence bibliographique rappelle tout d'abord l'importance sociale des affections allergiques, en particulier celles qui sont dues au pollen. Il existe de nombreuses plantes dont les floraisons accompagnées de production de pollen, provoquent des réactions allergiques pendant des périodes relativement longues de l'année. L'article en question ainsi que les publications mentionnées à la fin de celui-ci sont introduits à titre de référence dans la présente description.

A titre de documents illustrant la technique antérieure, on peut citer l'article cité dans Biological Abstracts, vol-66, 1er juillet 1978, ref. 71, page 76, PHila-PA (US) et Clin. Allergy 8 (1): 33–38, 1978, M.D. TOPPING et al.: «Allergenic activity of fractions of coksfoot (Dactylis glomerata) pollen», ainsi que le brevet US-A-1 613 313. Ces documents concernent des fractions protéiques extraites du pollen et leurs applications à titre de médicaments anti-allergiques. Les procédés de fractionnement décrits ne conduisent pas à la protéine (ou glycoprotéine) faisant l'objet de la présente invention, mais à des fractions protéiques ayant une haute activité allergénique (article de Biological Abstracts précité) ou à une fraction allergénique qui se combine avec IgE (brevet US-A-1 613 313). La technique antérieure ne révèle pas une fraction protéique permettant d'éviter une réaction allergénique par blocage de la synthèse des IgE.

La présente invention a pour objet une protéine possédant des propriétés immunologiques particulières. Cette protéine est utile dans le domaine de la lutte contre les affections et manifestations allergiques.

Ainsi, l'invention concerne un nouveau produit consistant en une 1-glycoprotéine présente dans le pollen de Dactylis glomerata, ayant un poids moléculaire de 14 000 daltons et un point isoélectrique de 4,6 environ, son spectre d'absorption présentant une absorption de $\varepsilon = 0,12$ unité de densité optique D.O.à 280 nm pour une solution aqueuse de 1 mg (poids sec) par ml d'eau.

L'invention a également pour objet un procédé permettant d'isoler ladite protéine à partir du pollen d'une graminée, le dactyle.

Ainsi, l'invention a encore pour objet un procédé pour l'obtention dudit nouveau produit protéique caractérisé en ce qu'on met en contact du pollen, en particulier de graminée, et de préférence du pollen de dactyle ou Dactylis glomerata avec un milieu aqueux, en ce qu'on recueille le surnageant, en ce qu'on soumet la fraction extraite soluble à un traitement de fractionnement et en ce qu'on isole la protéine ayant une masse molaires de 14 000 g environ (poids moléculaire: 14 000 daltons) et un point isoélectrique de 4,6 environ.

Le matériel végétal de départ dans le procédé de l'invention est avantageusement une graminée dénommée Dactylis glomerata. Le pollen est mis en contact avec un milieu aqueux de manière à fournir une fraction soluble. Le mode de réalisation le plus simple consiste à placer le pollen dans de l'eau et à laisser incuber jusqu'à ce que la fraction soluble soit extraite. On peut travailler avantageusement à la température ambiante. La durée d'incubation dépend du volume du milieu réactionel mis en œuvre. Dans la pratique, à température ambiante, des durées d'incubation de l'ordre de une heure se sont avérées convenables. Les quantités relatives du milieux aqueux, en particulier d'eau distillée et de pollen de dactyle peuvent varier dans de larges limites. Le poids de pollen proportionnellement au volume d'eau peut être de 10 à 200 parties en poids, par exemple de l'ordre de 100 parties en poids, par volume d'eau.

Une fois l'incubation terminée, on sépare le surnageant des parties solides encore présentes dans le milieu réactionnel et l'on procède alors au fractionnement de la fraction soluble, de manière à isoler la protéine ayant les caractéristiques définies précédemment.

Pour la caractérisation de la nouvelle protéine, on peut faire appel à la technique d'isoélectrofocalisation préparative décrite par RADOLA, B.J. (1973). Biochim. Biophys. Acta, 295, 412–428. Cette technique est connue de l'homme de l'art qui pourra, si nécessaire, se reporter à cette dernière référence bibliographique pour en connaître les détails d'exécution. La technique de fractionnement en question permet, en faisant varier le pH, de recueillir les fractions purifiées en couche mince de gel granuleux (Sephadex® ou Biorad®, par exemple). Lorsqu'on traite la fraction soluble extraite du pollen de dactyle, par la technique en cause, on constate la présence de plus de 60 bandes entre les pH 3,8 et 9. La protéine selon l'invention est séparée à pH 4,6.

En variante, on peut isoler la nouvelle protéine par la technique dite de chromatofocalisation, telle que décrite par exemple dans le fascicule dénommé «Chromatofocusing» distribué par Phar- macia Fine Chemicals AB Box 175 Uppsala Suède; voir également «Chromatofocusing»: isoelectric focusing on ion exchange columns pages 17–44 J. Chromatogr.150 (1978).

La protéine de l'invention possède des propriétés immunologique particulières grâce à son activité immunorégulatrice. En effet, au lieu d'induire la synthèse d'IgE et d'IgG homocytotropes (pour la définition de cette expression, voir par exemple page 1010 de l'ouvrage Immunologie de Paul Bordet (1972) Edit. Flammarion), immunoglobulines spécifiques de la réponse allergénique, elle inhibe fortement la synthèse de cette classe d'immunoglobulines et ne diminue que peu celle des IgA, IgM et autres IgG. Les études réalisées ont montré notamment que la nouvelle protéine était capable d'inhiber la synthèse d'IgE et d'IgG homocytotropes après sensibilisation d'un organisme receveur au pollen du dactyle. Ces résultats démontrent l'activité spécifique de la protéine immunorégulatrice selon l'invention.

La protéine selon l'invention est donc utile comme médicament des maladies allergiques en particulier des pollinoses. Ainsi, l'invention concerne un médicament contenant, à titre d'agent actif, la protéine ci-dessus définie. Plus particulièrement, l'invention concerne un médicament immunorégulateur de même qu'un médicament antiallergique contenant, à titre d'agent actif, ladite protéine.

L'invention a également pour objet des compositions pharmaceutiques contenant une quantité efficace de la nouvelle protéine en association avec un véhicule pharmaceutiquement acceptable et convenant à l'administration considérée. En vue de l'administration, diverses voies sont possibles, notamment la voie orale, parentérale par inhalation ou encore par injection. Il va sans dire que les véhicules devront être choisis différemment dans chacune de ces voies. Les formes galéniques peuvent être déterminées aisément par l'homme de l'art en fonction du mode d'administration choisi.

La nouvelle protéine de l'invention est très soluble dans l'eau. On dissout facilement 5 mg de protéine dans 1 ml d'eau. Elle se prête à la lyophilisation. La poudre lyophilisée conserve l'intégralité des propriétés de la poudre ordinaire.

L'homme de l'art comprendra donc que le produit de l'invention peut être utilisé dans des compositions pharmaceutiques très variées. Sans que les indications ci-après aient un caractère limitatif, on peut présenter les compositions sous forme de solutés injectables. Le produit peut aussi être présent sous forme de poudre sèche ou lyophilisée par exemple pour les préparations aptes à l'inhalation, notamment sous forme d'aérosol. Le produit solide peut aussi être adsorbé sur un adjuvant et être ainsi utilisé en injection. On peut aussi prévoir des compositions à base aqueuse ou huileuse pour des administrations par installations nasale ophtalmique ou orale, ou par inhalation. D'autres formes galéniques traditionnelles pour l'administration orale peuvent également convenir, telles que gélules, capsules, comprimés et autres formes analogues.

Les propriétés thérapeutiques de la nouvelle protéine s'exercent à des doses faibles et l'on a constaté aucun effet secondaire indésirable.

On notera que pour les applications envisagées, la protéine de l'invention possède un ensemble de propriétés très avantageuses. Elle est issue du fractionnement d'un produit d'origine naturelle, le pollen d'une graminée. Elle est active à faible dose. Sa toxicité est inexistante dans les conditions normales d'application. Ainsi, injectée à la dose de 100 µg/souris, soit 5 mg/kg de poids chez l'homme, on n'a trouvé aucune toxicité, alors que la dose la plus efficace se situe à des valeurs 1000 fois inférieures (0,1 µg au lieu de 100 µg chez la souris). Cette dernière propriété est très importante, car on connaît les dangers d'une utilisation d'allergènes non modifiés, par exemple d'un extrait total de pollen. Des accidents très sérieux, voire mortels, ont eu lieu dans le passé, lorsque de tels produits ont été injectés à des sujets souffrant d'allergie dans le cadre d'un traitement de désensibilisation.

Il est intéressant également que la protéine exerce un effet sélectif dans l'inhibition des anticorps formés lors des réactions allergiques. Le domaine préféré d'utilisation est celui de la lutte contre les réactions allergiques provoquées par les pollens de graminées.

Chez l'homme adulte une posologie convenable pour le traitement des pollinoses peut s'étendre de 1 µg à 5 mg, de préférence être de l'ordre de 0,01 mg, prendre en une seule injection.

Par voie orale, des doses jusqu'à 10 mg sont possibles. Par voie locale les doses admissibles peuvent aller jusqu'à 100 mg.

L'invention sera maintenant illustrée sans être aucunement limitées par la description ci-après qui donne un exemple d'isolement de la nouvelle protéine à partir du pollen de dactyle ainsi que les résultats de certaines études de pharmacologie.

Exemple

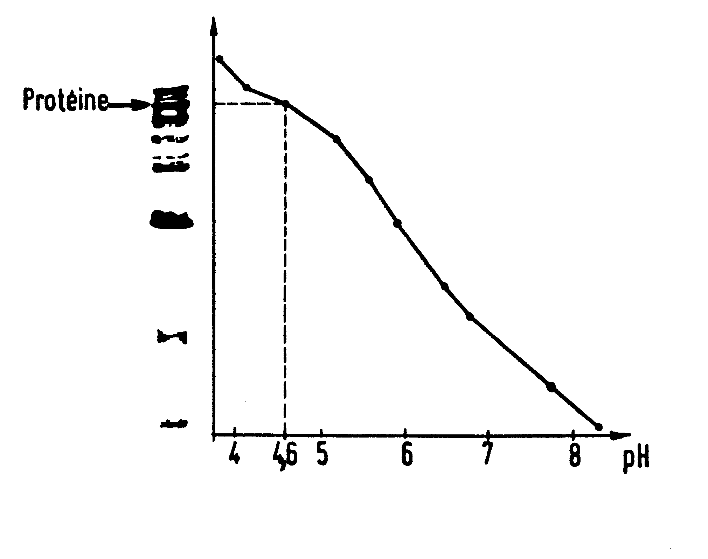

On utilise le pollen provenant de la graminée Dactylis glomerata. On incube 50 mg de ce pollen dans 0,5 ml d'eau distillée pendant une heure à température ambiante. On sépare le surnageant et l'on fractionne les constituants de la fraction soluble de pollen par la technique d'isoélectrofocalisation préparative (voir article de RADOLA précité). Par cette technique, entre les pH 3,8 et 9, la fraction soluble du pollen se sépare en plus de 60 bandes. On récupère la bande dont la masse molaire est de 14 000 g (poids moléculaire 14 000 daltons) et le point isoélectrique de 4,6. Les résultats de fractionnement sont illustrés à la figure 1 qui est un diagramme dans lequel on a porté en abscisses les valeurs du pH et en ordonnées les produits fractionnés. La protéine selon l'invention est isolée à pH 4,6.

La partie caractéristique du spectre d'absorption de la nouvelle protéine présente une absorption ε de 0,12 unité de D.O. à 280 nm pour une solution aqueuse de 1 mg (poids sec) par ml d'eau.

Dans une première série d'essais, on a évalué la réponse antipollen sur des animaux de laboratoire. Ceux-ci sont des souris femelles (Balb/c. Cette souche a été choisie parce qu'elle est une bonne répondeuse en IgE. Pour mesurer la quantité d'anticorps du type IgE, on a utilisé le test d'anaphylaxie cutanée passive, en abréviation PCA décrit par OVARY, Z., STEVEN, S., CAIAZZA and SOMEI KOPINA (1975). Int. Archs. Allergy Appln. Imm. $\underline{48}$, 16–21. Pour le dosage des anticorps de type IgM, IgG, IgA on a utilisé la technique communément dénommée test ELISA, abréviation de l'expression anglaise «Enzyme Linked Immunosorbent Assay», qui est notamment décrit dans l'article de ENGVALL, E. and PERLMANN, P. (1971) Immunochemistry, 8,871. Dans ce qui suit et par commodité, on désignera ces méthodes de mesure par leurs abréviations respectives PCA et ELISA.

De même, la fraction soluble totale de pollen de dactyle est désignée par l'abréviation FS. On a apprécié la réponse immunitaire de type IgE mesurée par PCA et de type non IgE mesurée par ELISA obtenue par deux injections par voie intrapéritonéale et à 21 jours d'invervalle de 10 µg de FS absorbés sur 1 mg de AlPO$_4$. Ce protocole d'immunisation s'est révélé optimal pour l'induction d'IgE antipollen de dactyle. Après les injections de FS aux jours 0 et 21, on a injecté la protéine (P) de l'invention au jour 28. Le tableau I se trouvant en fin de description rassemble les résultats obtenus. Le sous-tableau 1$\underline{a}$ concerne l'inhibition de la réponse antipollen de type IgE, le sous-tableau 1$\underline{b}$ est relatif à l'inhibition de la réponse antipollen de type IgG$_1$ homocytotropes, et le sous-tableau 1$\underline{c}$ illustre la réponse antipollen de type IgA, IgM et autres IgG.

Dans le sous-tableau 1$\underline{a}$, plus le titre révélé par PCA est élevé, plus concentration d'anticorps IgE anti-FS de pollen de dactyle est élevée. On voit que la protéine (P) à des doses de 1 µg/souris et 0,1 µg/souris inhibe la concentration d'IgE anti-FS de pollen de dactyle.

Dans les sous-tableau 1$\underline{b}$ et 1$\underline{c}$, plus le titre d'Ig mesuré par ELISA est élevé, plus la concentration d'anticorps de la classe considérée est élevée. On voit que la protéine (P) à des doses de 1 µg/souris et 0,1 µg/souris inhibe le taux d'IgG$_1$, anticorps homocytotropes (sous tableau 1$\underline{b}$) mais pas de façon significative le taux des anticorps de classes, IgA, IgM et les autres IgG (voir sous-tableau 1$\underline{c}$).

Ainsi, les résultats du tableau 1 montrent que l'injection de la protéine (P) en présence d'AlPO$_4$ à des souris ayant reçu 2 injections de FS à 10 µg induit une inhibition aussi bien de la réponse IgE que de la réponse IgG homocytotrope anti-FS. De plus, il n'y a pas d'inhibition significative des autres classes d'immunoglobulines. On constate aussi que la protéine de l'invention est efficace à des doses faibles.

On a également trouvé que la protéine (P) n'induit aucune réponse anticorps aux doses allant de 0,01 µg à 100 µg par souris même après deux injections de rappel. On constate donc que la protéine de l'invention est exempte d'effet secondaire indésirable dans de telles conditions d'utilisation.

Dans une deuxième série d'essais, on a évalué l'absence d'inhibition réalisée par la protéine de l'invention de la réaction allergique provoquée par une injection d'ovalbumine. Les animaux d'essais étaient du même type que ceux utilisés dans la première série. L'ovalbumine a été injectée à raison de 1 µg par souris au jour 0. La protéine (P) a été injectée le 15e jour. Les résultats obtenus sont rassemblés dans le tableau II qui suit.

Tableau I

| Inhibition de la production d'IgE anti-pollen de dactyle et d'IgG, A et M anti-pollen de dactyle par la protéine (P) de l'invention | | | | | | | | | |
|---|---|---|---|---|---|---|---|---|---|

1a – Inhibition de la réponse anti-pollen de type IgE par PCA chez la souris

| | (P) | | | | | | | | |
|---|---|---|---|---|---|---|---|---|---|
| | 0 | 7 | 14 | 21 | 28 | 35 | 42 | 49 | 56 | 63 |
| Test PCA après injection de (P) | FS 10 µg/ souris | | | FS 10 µg/souris | | | | | |
| 1 µg/s | | | | 0 | 270 | 30 | | 90 | | 0 |
| 0,1 µg/s | | | | 0 | 270 | 0 | | 10 | | 10 |
| 0,01 µg/s | | | | 0 | 270 | 90 | | 270 | | 270 |
| 0 µg/s | | | | 0 | 270 | 270 | | 270 | | 270 |

1b – Inhibition de la réponse anti-pollen du type IgG par ELISA (résultats exprimés en D.O. à 405 nm)

| Test ELISA après injection de (P) | 0 | 7 | 14 | 21 | (P) 28 | 35 | 42 | 49 | 56 | 63 |
|---|---|---|---|---|---|---|---|---|---|---|
| 1 µg/souris | | | | | 0,23 | 0,075 | 0,105 | 0,11 | 0,06 | 0,07 |
| 0,1 µg/souris | | | | | 0,23 | 0,1 | 0,15 | 0,18 | 0,08 | 0,1 |
| 0,01 µg/souris | | | | | 0,23 | 0,12 | 0,21 | 0,32 | 0,22 | 0,25 |
| 0 µg/souris | | | | | 0,23 | 0,25 | 0,25 | 0,27 | 0,21 | 0,24 |

1c – Réponse anti-pollen de type IgA, IgM et autres IgG

| Test ELISA après injection de (P) | 0 FS 10 µ/souris | 7 | 14 | 21 FS 10 µg/souris | (P) 28 | 35 | 42 | 49 | 56 | 63 |
|---|---|---|---|---|---|---|---|---|---|---|
| 1 µg/s | | | 0 | 0,15 | 0,47 | 0,47 | 0,35 | 0,41 | 0,39 | 0,33 |
| 0,1 µg/s | | | 0 | 0,15 | 0,47 | 0,48 | 0,37 | 0,42 | 0,33 | 0,27 |
| 0,01 µg/s | | | 0 | 0,15 | 0,47 | 0,49 | 0,35 | 0,40 | 0,33 | 0,32 |
| 0 µg/s | | | 0 | 0,15 | 0,47 | 0,43 | 0,46 | 0,40 | 0,41 | 0,31 |

Tableau II

| | (P) | | |
|---|---|---|---|
| 0 | 9 | 15 | 21 jours |
| OVA 1 µ/souris dans AlPO$_4$ | | | |
| (P) 1 µg/souris | 30 | 270 | 270 |
| (P) 0,1 µg/souris | 30 | 270 | 270 |
| 0 (témoin) | 30 | 270 | 270 |

Dans la présente description, on a dénommé «protéine», le produit de l'invention, ceci dans un but de signification. Cependant, le nouveau produit contient à la fois des acides aminés et des sucres et il constitue donc une glycoprotéine.

L'invention est encore illustrée par les essais cliniques suivants.

Trente et un malades sensibles au pollen de graminées, 14 hommes et 17 femmes ont été traités en mai et juin 1982 par une seule injection souscutanée de 5 µg de la nouvelle protéine en solution physiologique. Treize malades ont été traités entre 3 et 1 semaines avant le début de la saison pollinique (en mai), 18 l'ont été entre 1 à 3 semaines après le début de la saison pollinique (en juin). Vingt trois malades ont déclaré avoir été améliorés par rapport aux saisons polliniques précédentes. La mesure de cette amélioration à été suivie par l'absence de prise de médicaments anti-allergie chez les malades traités en mai et surtout par la baisse de consommation de ces médicaments chez les malades traités au début ou au cours de la saison pollinique. Cette baisse a été régulière au cours des 5 premiers jours suivant le traitement conduisant à l'arrêt de la prise de médicaments anti-histaminiques ou cromoglycate de sodium une semaine après le traitement.

Ces essais cliniques montrent l'utilité du produit selon l'invention pour traiter les pollinoses.

**Revendications**

1. 1-Glycoprotéine présente dans le pollen de Dactylis glomerata, ayant un poids moléculaire de 14 000 daltons et un point isoélectrique de 4,6 environ, son spectre d'absorption présentant une absorption de $\varepsilon = 0,12$ unité de densité optique D.O. à 280 nm pour une solution aqueuse de 1 mg (poids sec) par ml d'eau.

2. Procédé pour l'obtention de la 1-glycoprotéine selon la revendication 1, caractérisé en ce qu'on met en contact du pollen, en particulier de graminée, et de préférence du pollen de dactyle ou Dactylis glomerata avec un milieu aqueux, en ce qu'on recueille le surnageant, et en ce qu'on soumet la fraction extraite soluble à un traitement de fractionnement et en ce qu'on isole la protéine ayant un poids moléculaire de 14 000 daltons environ et un point isoélectrique de 4,6 environ.

3. Procédé selon la revendication 2, caractérisé en ce qu'on incube le pollen dans l'eau distillée.

4. Procédé selon l'une des revendications 2 ou 3, caractérisé en ce qu'on opère à la température ambiante.

5. Procédé selon l'une quelconque des revendications 2 à 4, caractérisé en ce qu'on réalise le fractionnement de la fraction soluble (F.S.) par la technique d'isoélectrofocalisation ou de chromatofocalisation préparative.

6. Médicament contenant, à titre d'agent actif, la 1-glycoprotéine selon la revendication 1.

7. Médicament immunorégulateur selon la revendication 6.

8. Médicament antiallergique selon la revendication 6.

9. Médicament selon l'une quelconque des revendications 6 à 8, caractérisé en ce qu'il induit l'inhibition de la synthèse des IgE et IgG homocytotropes.

10. Composition pharmaceutique contenant une quantité efficace de la 1-glycoprotéine selon la revendication 1, en association avec un véhicule pharmaceutiquement acceptable adapté à l'administration orale, parentérale, par injection ou par inhalation.

## Patentansprüche

1. 1-Glycoprotein, vorkommend im Blütenstaub von Dactylis glomerata, mit einem Molekulargewicht von 14 000 Dalton und einem isoelektrischen Punkt von etwa 4,6, dessen Absorptionsspektrum eine Absorption von $\varepsilon = 0,12$ Einheiten der optischen Dichte bei 280 nm für eine wässrige Lösung von 1 mg (Trockengewicht) pro ml Wasser zeigt.

2. Verfahren zur Herstellung von 1-Glycoprotein gemäss Anspruch 1, dadurch gekennzeichnet, dass man Blütenstaub, insbesondere die Halme und vorzugsweise den Blütenstaub der Dactylis oder Dactylis glomerata mit einem wässrigen Milieu in Kontakt bringt, das an der Oberfläche schwimmende einsammelt und die lösliche Extraktionsfraktion einer Fraktionierung unterwirft und das Protein, das ein Molekulargewicht von etwa 14 000 Dalton und einen isoelektrischen Punkt von etwa 4,6 besitzt, isoliert.

3. Verfahren gemäss Anspruch 2, dadurch gekennzeichnet, dass man den Blütenstaub in destilliertes Wasser einbringt.

4. Verfahren gemäss einem der Ansprüche 2 oder 3, dadurch gekennzeichnet, dass man bei Raumtemperatur arbeitet.

5. Verfahren gemäss irgendeinem der Ansprüche 2 bis 4, dadurch gekennzeichnet, dass man die Fraktionierung der löslichen Fraktion durch Techniken der präparativen isoelektrischen Fokussierung oder Chromatofokussierung verwirklicht.

6. Medikament, enthaltend als aktives Agens das 1-Glycoprotein gemäss Anspruch 1.

7. Immunoregulatives Medikament gemäss Anspruch 6.

8. Antiallergenes Medikament gemäss Anspruch 6.

9. Medikament gemäss irgendeinem der Ansprüche 6 bis 8, dadurch gekennzeichnet, dass die Inhibierung der Synthese der homocytotropen IgE und IgG induziert wird.

10. Pharmazeutische Zusammensetzung, enthaltend eine wirksame Menge des 1-Glycoproteins gemäss Anspruch 1 in Verbindung mit einem pharmazeutischen Träger, der für die orale, parenterale, durch Injektion oder durch Inhalation durchzuführende Verabreichung angepasst ist.

## Claims

1. 1-Glycoprotein present in the pollen of Dactylis glomerata and having a molecular weight of 14,000 daltons and an isoelectric point of approximately 4.6, its absorption spectrum showing an absorption of $\varepsilon = 0.12$ optical density O.D. unit at 280 nm for an aqueous solution of 1 mg (dry weight) per ml of water.

2. Method for obtaining the 1-glycoprotein according to Claim 1, characterized in that pollen, especially pollen of graminaceae and preferably cocksfoot or Dactylis glomerata pollen, is brought into contact with an aqueous medium, in that the supernatant is collected, and in that the soluble extracted fraction is subjected to a fractionation treatment and in that the protein having a molecular weight of approximately 14,000 daltons and an isoelectric point of approximately 4.6 is isolated.

3. Method according to Claim 2, characterized in that the pollen is incubated in distilled water.

4. Method according to one of Claims 2 and 3, characterized in that it is performed at room temperature.

5. Method according to any one of Claims 2 to 4, characterized in that the fractionation of the soluble fraction (S.F.) is carried out by the technique of preparative isoelectric focusing or chromatofocusing.

6. Drug containing, as active agent, the 1-glycoprotein according to Claim 1.

7. Immunoregulatory drug according to Claim 6.

8. Antiallergic drug according to Claim 6.

9. Drug according to any one of Claims 6 to 8, characterized in that it induces inhibition of the synthesis of the homocytotropic IgE and IgG.

10. Pharmaceutical composition containing an effective amount of the 1-glycoprotein according to Claim 1, in combination with a pharmaceutically acceptable vehicle suited to oral or parenteral administration or administration by injection or inhalation.

1/1

Protéine ➡

7